# EUROPEAN PATENT APPLICATION

(11) **EP 3 626 328 A1**
(43) Date of publication of application: **25.03.2020**
(21) Application number: 19197151.4
(22) Date of filing: 13.09.2019
(51) Int. Cl.: B01D 53/047, B01D 53/14, B01D 53/22, C12M 1/00, C01B 3/50

(54) **METHOD FOR SEPARATING A GAS MIXTURE BY PRESSURE SWING ADSORPTION**

(30) Priority: 20.09.2018 NL 2021677
(71) Applicant: Green Vision Holding B.V., 6827 AV Arnhem (NL)
(72) Inventor: DER KINDEREN, Joannes Maria, 7391 SJ Twello (NL); OUDENHOVEN, Tom Martin Jacob, 6831 MH Arnhem (NL); DOEVEN, Carolina Paulina, 6841 AK Arnhem (NL); WESTENDORP, Gerard, 6671 BL Zetten (NL)
(74) Representative: van Dam, Vincent

(57) **Abstract**

The invention relates to the purification of a gas that can be used as fuel starting from a crude gas mixture. A high purity level in fuel is of great importance to the combustion efficiency and moreover contributes to the life span of the constituent parts of machines in which the fuels are being used. The invention thus relates to a method for purifying a gas that can be used as fuel, by separating a first gas mixture (A/B)₁ of a first gas (A) that can be used as fuel and at least a second gas (B) into a first gas (A) and at least a second gas (B).

## Description

The invention relates to the purification of a gas that can be used as fuel starting from a crude gas mixture. A high purity level of fuel is of great importance to the combustion efficiency and moreover contributes to the life span of the parts of machines in which the fuels are being used. The invention thus relates to a method for purifying a gas that can be used as fuel, by separating a first gas mixture (A/B)₁ of a first gas (A) that can be used as fuel and at least a second gas (B), into a first gas (A) and at least a second gas (B), which method comprises the steps of (i) providing the first gas mixture (A/B)₁, (ii) separating the first gas mixture (A/B)₁ into a second gas mixture (A/B)₂ of the first gas (A) and the second gas (B), in which the concentration [A]₂ of the first gas (A) is higher than the concentration [A]₁ of said gas in the first gas mixture (A/B)₁, wherein the pressure of the second gas mixture (A/B)₂ has a first value *p*₁, and into a third gas mixture (A/B)₃ of the first gas (A) and the second gas (B), in which the concentration [B]₃ of the second gas (B) is higher than the concentration [B]₁ of said gas in the first gas mixture (A/B)₁, and (iii) separating the second gas mixture (A/B)₂ into a fourth gas mixture (A/B)₄ of the first gas (A) and the second gas (B), in which the concentration [A]₄ of the first gas (A) is higher than the concentration [A]₂ of said gas in the second gas mixture (A/B)₂, and into a residual gas mixture (A/B)₅ of the first gas (A) and the second gas (B), in which the concentration [B]₅ of the second gas (B) is higher than the concentration [B]₂ of said gas in the second gas mixture (A/B)₂, which step (iii) is carried out according to a pressure swing adsorption process (PSA process), wherein the second gas mixture (A/B)₂ is introduced into at least one vessel which contains an adsorption mass for adsorbing at least the second gas (B) .

Separating a gas mixture according to a PSA process in a first separation device is known per se. The method is for instance used to separate hydrogen gas of extremely high purity (99,999%) from a so-called reformate, a mixture of hydrogen gas (H₂), carbon dioxide (CO₂), carbon monoxide (CO) and methane (CH₄).

When separating a gas mixture of a first gas (A) and at least a second gas (B) by means of a PSA process, use is made of an adsorption mass of a selective adsorbent, wherein the adsorption strength for the first gas (A) is lower than the adsorption strength for the second gas (B). In that way the second gas is adsorbed to the adsorption mass in the respective vessel in question at a relatively high pressure, for instance 10 bar, following which the first gas, that has not been adsorbed, can be released from the vessel.

The adsorption mass is periodically regenerated, for which purpose a purge gas containing a substantially pure product gas is introduced into the respective vessel at a relatively low pressure, for instance 0.2 bar. The required use of product gas as purge gas for regenerating the adsorption mass, results in an upper limit to the value of the yield of product gas that can be realized with a separation according to the PSA process. The yield, that means the ratio of the quantities of product gas obtained and the product gas used for regenerating the adsorption mass in a first order approximation corresponds to the ratio of the pressure of the gas mixture introduced into the PSA device and the pressure of the purge gas.

It is an object of the invention to provide a method for purifying gas that can be used as fuel starting from a crude gas mixture wherein the upper limit of the yield of a product gas that can be used as fuel is increased, while maintaining the demands of purity set for the product gas.

This objective is achieved, and other advantages are realized using a method of the type mentioned in the preamble, wherein in accordance with the invention the value of the pressure (*p*₁) of the second gas mixture (A/B)₂ is increased to a value *p*₂ (*p*₂>*p*₁) prior to introducing it into the at least one vessel.

Separating a gas mixture at a high pressure in a PSA device according to the state of the art is usually considered undesirable, because at a high pressure the partial pressures of the (pollution) gases to be separated are relatively high, as a result of which the adsorbent may become saturated.

In a method in accordance with the invention, wherein a PSA process is applied once a first separation has taken place in a first separation device, the concentration of the pollutants has been reduced to such an extent that even at the very high overall pressure, the partial pressures of the pollutants in the PSA device do not saturate the material of the adsorption mass. The gas released in the PSA device during purging can be passed back to the first separation device, and as a consequence does not result in a loss of product gas. That is why according to the method according to the invention the residual gas mixture (A/B)₅ is added to the first gas mixture (A/B)₁ to be separated according to step (ii). Adding the residual gas mixture (A/B)₅ to the first gas mixture (A/B)₁ makes it possible to realize an exceptionally high yield of the separation process.

The value of *p*₁ is for instance increased by a factor of at least 10 to a value *p*₂ (*p*₂ ≥ 10 *p*₁), preferably by a factor of at least 30 (*p*₂ ≥30 *p*₁), more preferably by a factor of at least 50 (*p*₂ ≥ 50 *p*₁).

In a practical situation, in which the second gas mixture is passed at a first pressure with a value *p*₁ = 6 bar to the second PSA separation device, the pressure of said gas is increased to a value *p*₂ amounting to for instance 60 bar, preferably 180 bar, more preferably 300 bar. If in said second gas mixture the concentration of the gas to be separated is for instance lower than 0.1%, the partial pressure of said gas at an overall pressure of 300 bar is 0.3 bar, and the adsorbent in the PSA device will not be saturated with said gas.

The yield of a separation of a gas mixture which is introduced into an PSA device at a relatively very high pressure of for instance 300 bar and which is purged using a product gas at a relatively low pressure of for instance 0.2 bar, is very high (approximately 1500 with the exemplary values mentioned) compared to the yield of a separation in a PSA device according to the state of the art (in which the yield typically is between the values 8 and 20).

In one embodiment of a method according to the invention, the step (ii) of separating the first gas mixture (A/B)₁ is carried out according to a washing process. The separation according to a washing process for instance is of importance in the separation of gas mixtures that are released during fermenting a biomass.

In another embodiment that for instance is of importance in the separation of gas mixtures that are released during fermenting a biomass, the step (ii) of separating the first gas mixture (A/B)₁ is carried out by means of a membrane.

In one embodiment the invention relates to the separation of a gas containing methane. Crude gas mixtures originating from accepted gassing processes such as biogas or reform gas, usually contain methane that on the one hand may serve as combustible material and on the other hand may be a pollutant if the crude gas mixture comprises another combustible gas of interest.

In one embodiment methane is the gas of interest and it is of importance that the product gas contains methane of high purity. In this embodiment the method is therefore aimed at the purification of methane. Gas (A) in the first gas mixture (A/B)₁ can then comprise or be methane (CH₄). Gas mixtures containing crude methane, which mixtures are obtained through converting a biomass according to a gassing process, usually contain pollutants in the form of carbon dioxide (CO₂). The first gas mixture (A/B)₁ can therefore at least also comprise carbon dioxide (CO₂). Gas (A) in the first gas mixture (A/B)₁ can then comprise methane (CH₄) and the gas (B) can comprise at least carbon dioxide (CO₂).

In another embodiment the initial gas (A/B)₁ comprises hydrogen (H₂) and the method is aimed at the purification of hydrogen. Gas (A) in the first gas mixture (A/B)₁ can then comprise or be hydrogen (H₂). The initial gas (A/B)₁ according to step (i) can in this case for instance be provided by means of converting a biomass according to a gassing process or by means of converting a hydrocarbon-containing gas mixture according to a steam reforming process. Apart from hydrogen (H₂) the crude initial mixture in these cases also at least contains methane, carbon monoxide (CO) and carbon dioxide (CO₂). In case of the purification of hydrogen, the first gas mixture (A/B)₁ can therefore at least comprise methane (CH₄), hydrogen (H₂), carbon monoxide (CO) and carbon dioxide (CO₂). Gas (A) in the first gas mixture (A/B)₁ then comprises hydrogen (H₂) and the gas (B) comprises at least one of the gases methane (CH₄), carbon monoxide (CO) and carbon dioxide (CO₂).

The present invention is aimed at the purification of a gas that can be used as fuel, or in other words a combustible gas or fuel gas. Both purified methane and hydrogen can be utilized as fuel, for instance in various industrial processes.

In another embodiment of a method according to the invention, the step (ii) of separating the first gas mixture (A/B)₁ is carried out according to a pressure swing process (PSA process). According to this embodiment both the first gas mixture (A/B)₁ and the second gas mixture (A/B)₂ are separated according to a PSA process, wherein the pressure of the second gas mixture (A/B)₂ obtained from the first separation process is increased prior to it being subjected to the next PSA process.

The gas (A) in the first gas mixture (A/B)₁ then for instance comprises hydrogen (H₂) and the gas (B) at least contains one of the gases methane (CH₄), carbon monoxide (CO) and carbon dioxide (CO₂), and the first gas mixture according to step (i) is for instance provided by means of converting a biomass according to a gassing process.

In a next example of the method according to the invention wherein both the first gas mixture (A/B)₁ and the second gas mixture (A/B)₂ are separated according to a PSA process, the gas (A) in the first gas mixture (A/B)₁ comprises hydrogen (H₂), and the gas (B) contains at least one of the gases methane (CH₄), carbon monoxide (CO) and carbon dioxide (CO₂), which first gas mixture according to step (i) is provided by means of converting a hydrocarbon-containing gas mixture according to a steam reforming process.

The method according to the invention can be carried out by means of a device for the separation of a first gas mixture (A/B)₁ of a first gas (A) and at least a second gas (B) into a first gas (A) and at least a second gas (B), comprising a first separation device for separating the first gas mixture (A/B)₁ into a second gas mixture (A/B)₂ of the first gas (A) and the second gas (B), in which the concentration [A]₂ of the first gas (A) is higher than the concentration [A]₁ of said gas in the first gas mixture (A/B)₁ wherein the pressure of the second gas mixture (A/B)₂ has a first value *p*₁, and into a third gas mixture (A/B)₃ of the first gas (A) and the second gas (B), in which the concentration [B]₃ of the second gas (B) is higher than the concentration [B]₁ of said gas in the first gas mixture (A/B)₁, a second separation device for separating the second gas mixture (A/B)₂ according to a pressure swing adsorption process (PSA process) into a fourth gas mixture (A/B)₄ of the first gas (A) and the second gas (B), in which the concentration [A]₄ of the first gas (A) is higher than the concentration [A]₂ of the first gas (A) in the second gas mixture (A/B)₂, and into a fifth gas mixture (A/B)₅ of the first gas (A) and the second gas (B), in which the concentration [B]₅ of the second gas (B) is higher than the concentration [B]₂ of said gas in the second gas mixture (A/B)₂, comprising a number of vessels, wherein each vessel has at least one inlet and one outlet, and an adsorption mass is provided in each of these vessels for adsorbing the second gas (B), wherein the at least one inlet is coupled to compressor means for increasing the pressure of the second gas mixture (A/B)₂ to a value *p*₂ (*p*₂>*p*₁) prior to introducing said second gas mixture (A/B)₂ into the at least one vessel.

In an advantageous embodiment the outlet of the at least one vessel is coupled to the inlet of the first separation device.

In one embodiment, the first separation device comprises a washing device.

In another embodiment, the first separation device comprises a membrane.

In yet another embodiment, the first separation device comprises a device for a pressure swing adsorption process (PSA process), which device comprises a number of vessels, wherein each vessel has at least one inlet and one outlet and an adsorption mass is provided in each of these vessels for adsorbing the second gas (B).

A device according to this embodiment is particularly suitable for separating hydrogen (H₂) from a first gas mixture containing at least one of the gases carbon monoxide (CO), carbon dioxide (CO₂) and methane (CH₄), which first gas mixture is obtained by means of converting a hydrocarbon-containing gas mixture according to a steam reforming process.

In a device according to the latter embodiment which is particularly suitable for separating a first gas mixture (A/B)₁ produced in a gassing device of a biomass, the at least one inlet is coupled to compressor means for increasing the pressure of the first gas mixture (A/B)₁ prior to being introduced into the at least one vessel.

The invention will be elucidated below on the basis of exemplary embodiments, while referring to the drawings.

The drawings show schematic views in which:
Figure 1 is a production device for a gas mixture (A/B) that is coupled to a first embodiment of a separation device according to the invention,
Figure 2 is a device for fermenting biomass that is coupled to a second embodiment of a separation device according to the invention,
Figure 3 is a device for fermenting biomass that is coupled to a third embodiment of a separation device according to the invention,
Figure 4 is a device for gassing biomass that is coupled to a fourth embodiment of a separation device according to the invention, and
Figure 5 is a device for generating a hydrogencontaining gas mixture that is coupled to a fifth embodiment of a separation device according to the invention.

In the figures, similar parts are referred to by the same reference numbers.

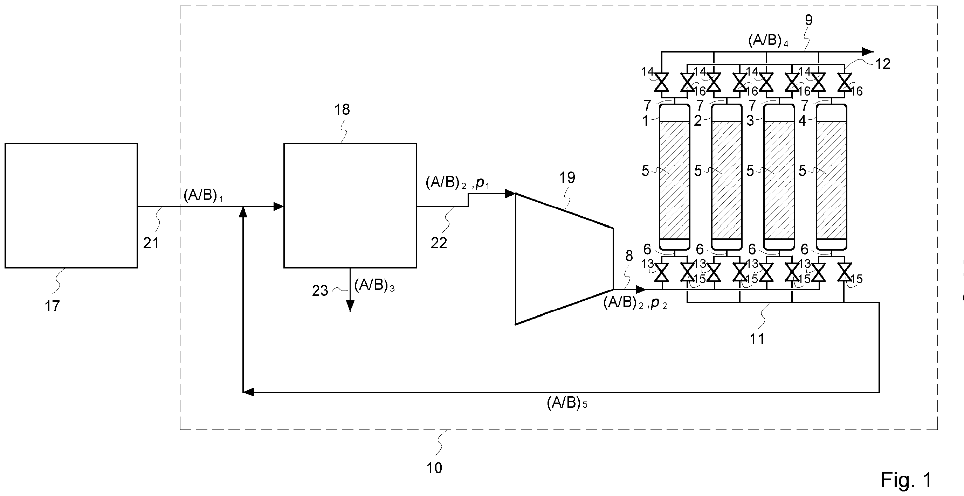

Figure 1 schematically shows a production device 17 for the production of a first gas (A), wherein a first gas mixture (A/B)₁ of a first gas (A) and at least a second gas (B) is obtained. The production device 17 is coupled to a separation device 10 by a conduit, which separation device comprises an assembly of, connected in series, a first separation device 18, a compressor 19 and a PSA device which in this example has four adsorption vessels 1, 2, 3, 4. The first separation device 18 is configured for separating the first gas mixture (A/B)₁ into a second gas mixture (A/B)₂ of the first gas (A) and the second gas (B), in which the concentration [A]₂ of the first gas (A) is higher than the concentration [A]₁ of said gas in the first gas mixture (A/B)₁, wherein the pressure of the second gas mixture (A/B)₂ has a first value *p₁,* and into a third gas mixture (A/B)₃ of the first gas (A) and the second gas (B), in which the concentration [B]₃ of the second gas (B) is higher than the concentration [B]₁ of said gas (B) in the first gas mixture (A/B)₁, a second separation device for according to a pressure swing adsorption process (PSA process) separating the second gas mixture (A/B)₂ into a fourth gas mixture (A/B)₄ of the first gas (A) and the second gas (B), comprising a number of vessels 1, 2, 3, 4. Each of the vessels 1, 2, 3, 4 is provided with an adsorption bed having an adsorption mass 5 of a material for having the second gas (B) adsorb to it, and with an inlet 6 and an outlet 7. The vessels 1, 2, 3, 4 are connected to four parallel conduits, namely a manifold 8 for the second gas mixture (A/B)₂ to be separated, a collecting conduit 9 for the fourth gas mixture (the product gas, (A/B)₄), a collecting conduit 11 for the separated fifth gas mixture (the exhaust gas, (A/B)₅), and a manifold 12 for equalization and purge gas. In each of the conduits 8, 9, 11, 12 a supply valve 13, a product valve 14, an exhaust gas valve 15 and an equalization and purge valve 16, respectively, are present for each of the vessels 1, 2, 3, 4. Because the second gas mixture (the feed gas, (A/B)₂) can be introduced into the vessels 1, 2, 3, 4 at a very high pressure *p₂,* an extremely high purity of the fourth gas mixture (the product gas, (A/B)₄) can be realized, that means a gas having a relatively extremely low concentration [B] of the second gas (B). As the exhaust gas (the fifth gas mixture (A/B)₅) is passed back from the PSA device to the first separation device 18 via the collecting conduit 11, an extremely high yield of the separation process can moreover be realized.

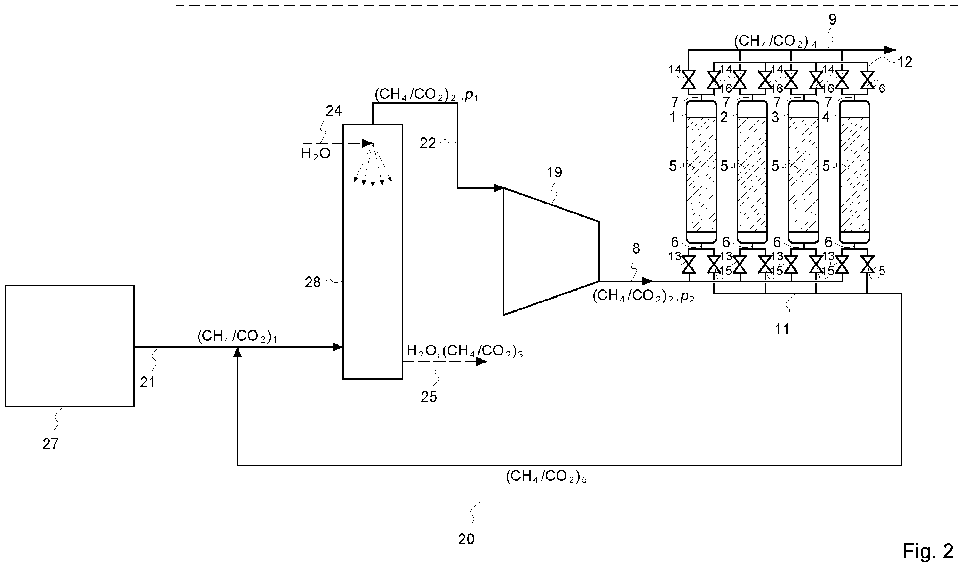

Figure 2 is a schematic view of a biomass fermenting device 27 for the production of methane (CH₄), wherein a first gas mixture (CH₄/CO₂)₁ of methane (the first gas (A)) and carbon dioxide (CO₂) (the second gas (B)) is obtained. The fermenting device 27 is coupled to a separation device 20 by a conduit 21, which separation device comprises an assembly of, connected in series, a washing tower 28, a compressor 19 and a PSA device which in this example has four vessels 1, 2, 3, 4. In the washing tower 28 the CO₂ in the supplied first gas mixture (CH₄/CO₂)₁ is washed out using a liquid, for instance a water-based solution of an amine-complex, which is introduced via inlet 24 and is sprayed, after which a methane-rich second gas mixture (CH₄/CO₂)₂ of methane and carbon dioxide is guided via conduit 22 to a compressor 19 at a pressure *p₁,* and a methane-poor third gas mixture (CH₄/CO₂)₃ of methane and carbon dioxide, dissolved in the liquid, is discharged via an outlet 25. By means of the compressor 19 the pressure of the methane-rich second gas mixture (CH₄/CO₂)₂ is increased from a value *p*₁ to a value *p*₂, after which this second gas mixture is introduced into the respective adsorption vessels 1, 2, 3, 4 of the PSA device, in which the gas mixture is further separated into highly pure product gas (CH₄/CO₂)₄ methane, with traces of carbon dioxide, which becomes available via collecting conduit 9, and a residual gas mixture (CH₄/CO₂)₅, which is passed back to the washing tower 28 via collecting conduit 11.

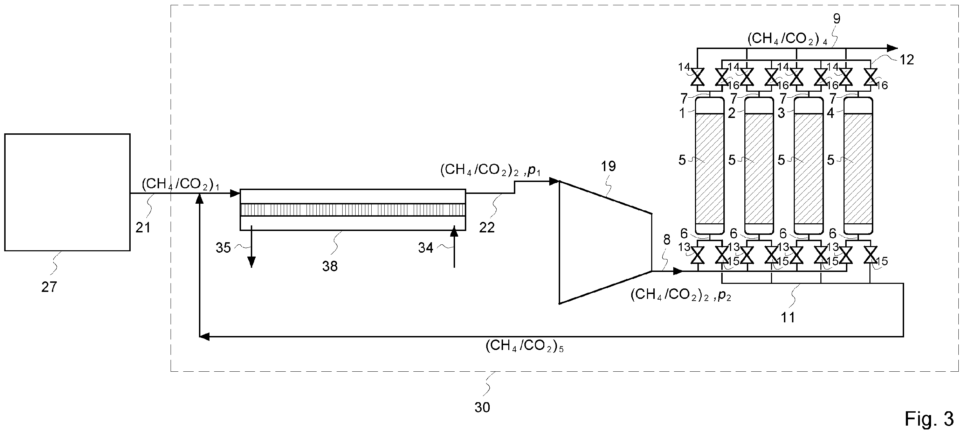

Figure 3 is a schematic view of a biomass fermenting device 37 for the production of methane (CH₄), that is coupled to a separation device 30 by a conduit 21, which separation device comprises an assembly of, connected in series, a membrane 28, a compressor 19 and a PSA device which in this example has four vessels 1, 2, 3, 4. In the membrane 38 the CO₂ in the supplied first gas mixture (CH₄/CO₂)₁ of methane and carbon dioxide, is dissolved in a fluid, which is introduced via inlet 34, after which a methane-rich second gas mixture (CH₄/CO₂)₂ of methane and carbon dioxide is guided via conduit 22 to a compressor 19 at a pressure *p*₁, and a methane-poor third gas mixture (CH₄/CO₂)₃ of methane and carbon dioxide is discharged via an outlet 35. The separation device 30 differs from the device 20 shown in Figure 2 in the use of a membrane 38 instead of a washing tower 28, but is otherwise identical to it.

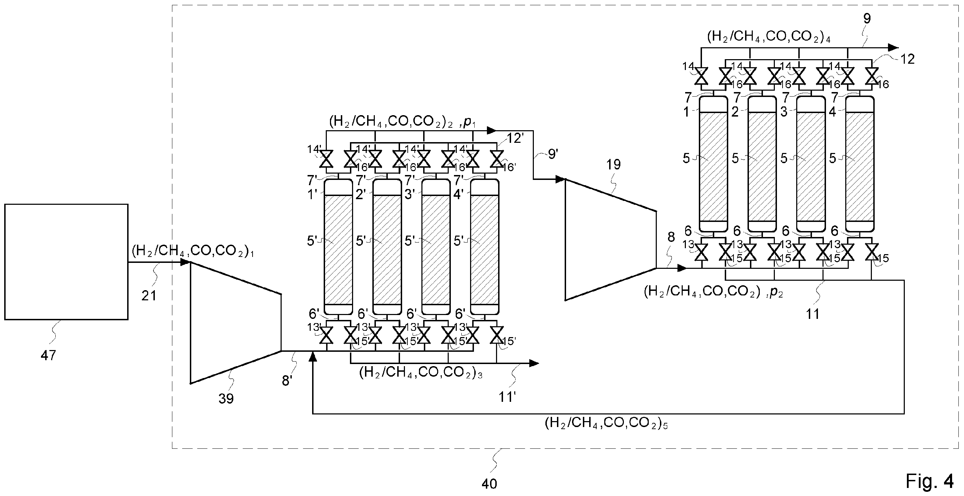

Figure 4 is a schematic view of a biomass gassing device 47 for the production of methane (CH₄), that is coupled to a separation device 40 by a conduit 21, which separation device comprises an assembly of, connected in series, a first compressor 39, a first PSA device which in this example has four adsorption vessels 1', 2', 3', 4', a second compressor 19 and a second PSA device which in this example has four vessels 1, 2, 3, 4. In the gassing device 47 a biomass accommodated therein is heated as a result of which said biomass is partially converted into a first gas mixture (H₂/CH₄, CO, CO₂)₁ containing hydrogen and at least one of the gases methane (CH₄), carbon monoxide (CO) and carbon dioxide (CO₂). By means of the first compressor 39 this first gas mixture is pressurized to for instance 8 bar, and introduced into the first PSA device 1', 2', 3', 4', where it is separated into a hydrogen-rich second gas mixture (H₂/CH₄, CO, CO₂)₂ of hydrogen, methane, carbon monoxide and carbon dioxide at a pressure *p₁,* which is guided to the second compressor 19 via a collecting conduit 9', and a hydrogen-poor third gas mixture (H₂/CH₄, CO, CO₂)₃ of hydrogen, methane, carbon monoxide and carbon dioxide, that is discharged via a collecting conduit 11'. By means of the compressor 19 the pressure of the hydrogen-rich second gas mixture is increased from a value *p*₁ to a value *p*₂ of for instance 60 bar, after which this second gas mixture is introduced into the respective adsorption vessels 1, 2, 3, 4 of the second PSA device, in which the gas mixture is further separated into highly pure product gas hydrogen, which becomes available via collecting conduit 9, and a residual gas mixture (H₂/CH₄, CO, CO₂)₅ having traces of methane, carbon monoxide and carbon dioxide, which is passed back to the first PSA device 1', 2', 3', 4' via collecting conduit 11.

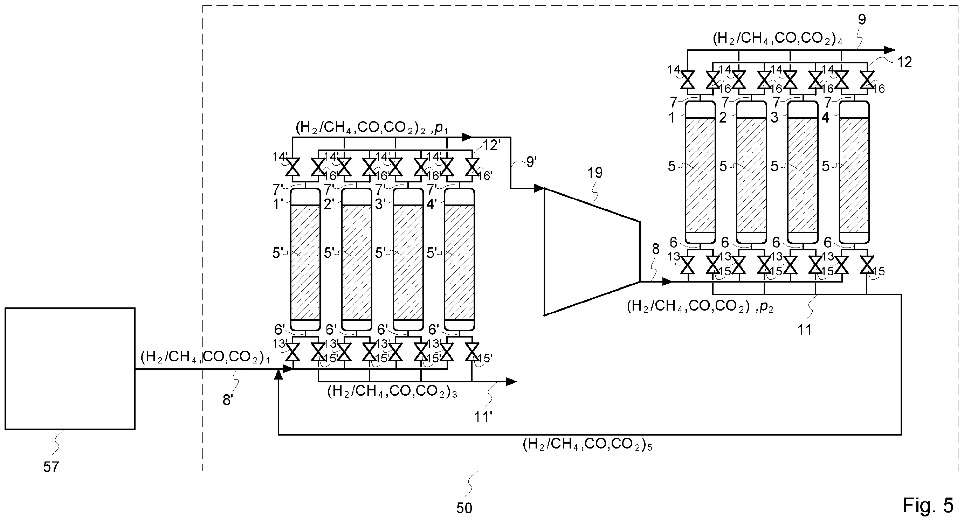

Figure 5 is a schematic view of a steam reformer 57 for the production of hydrogen (H₂), that is coupled to a separation device 50 by a conduit 8', which separation device comprises an assembly of, connected in series, a first PSA device which in this example has four adsorption vessels 1', 2', 3', 4', a compressor 19 and a second PSA device which in this example has four vessels 1, 2, 3, 4. In the steam reformer 57 an introduced mixture of methane (CH₄) and steam (H₂O) is heated and thus converted into a first gas mixture (H₂/CH₄, CO, CO₂)₁ containing hydrogen and at least one of the gases methane (CH₄), carbon monoxide (CO) and carbon dioxide (CO₂). The separation device 50 differs from the device 40 shown in Figure 4 in the absence of a first compressor, preceding the first PSA device, but is otherwise identical to it. The first gas mixture (H₂/CH₄, CO, CO₂)₁ is directly introduced into the first PSA device 1', 2', 3', 4', after which the separation takes place analogous to the manner as described for Figure 4 above.

## Claims

1. Method for purifying a gas that can be used as fuel, comprising separating a first gas mixture (A/B)₁ of a first gas (A) and at least a second gas (B) into a first gas (A) that can be used as fuel and at least a second gas (B), which method comprises the steps of:
(i) providing the first gas mixture (A/B)₁,
(ii) separating the first gas mixture (A/B)₁ into a second gas mixture (A/B)₂ of the first gas (A) and the second gas (B), in which the concentration [A]₂ of the first gas (A) is higher than the concentration [A]₁ of said gas in the first gas mixture (A/B)₁, wherein the pressure of the second gas mixture (A/B)₂ has a first value *p*₁, and into a third gas mixture (A/B)₃ of the first gas (A) and the second gas (B), in which the concentration [B]₃ of the second gas (B) is higher than the concentration [B]₁ of the second gas (B) in the first gas mixture (A/B)₁, and
(iii) separating the second gas mixture (A/B)₂ into a fourth gas mixture (A/B)₄ of the first gas (A) and the second gas (B), in which the concentration [A]₄ of the first gas (A) is higher than the concentration [A]₂ of the first gas (A) in the second gas mixture (A/B)₂, and into a residual gas mixture (A/B)₅ of the first gas (A) and the second gas (B), in which the concentration [B]₅ of the second gas (B) is higher than the concentration [B]₂ of the second gas (B) in the second gas mixture (A/B)₂, which step (iii) is carried out according to a pressure swing adsorption process (PSA process), wherein the second gas mixture (A/B)₂ is introduced into at least one vessel (1, 2, 3, 4) which contains an adsorption mass (5) for adsorbing at least the second gas (B),
wherein prior to introduction into the at least one vessel (1, 2, 3, 4) the value of the pressure (*p*₁) of the second gas mixture (A/B)₂ is increased to a value *p*₂ (*p*₂>*p*₁), and
wherein the residual gas mixture (A/B)₅ is added to the first gas mixture (A/B)₁ which is to be separated according to step (ii).

2. Method according to claim 1, wherein the first gas mixture (A/B)₁ contains methane (CH₄).

3. Method according to claim 1 or 2, wherein the gas (A) is or comprises methane.

4. Method according to any one of the claims 1 - 3, wherein the first gas mixture (A/B)₁ comprises at least methane (CH₄) and carbon dioxide (CO₂), and wherein gas (A) in the first gas mixture (A/B)₁ comprises methane (CH₄) and the gas (B) comprises at least carbon dioxide (CO₂).

5. Method according to claim 4, wherein the first gas mixture according to step (i) is provided by means of converting a biomass according to a gassing process.

6. Method according to claim 1 or 2, wherein gas (A) is or comprises hydrogen.

7. Method according to claim 6, wherein the first gas mixture (A/B)₁ comprises at least hydrogen (H₂), carbon monoxide (CO) and carbon dioxide (CO₂), and wherein gas (A) in the first gas mixture (A/B)₁ comprises hydrogen (H₂) and the gas (B) contains at least one of the gases methane (CH₄), carbon monoxide (CO) and carbon dioxide (CO₂).

8. Method according to claim 7, wherein the first gas mixture according to step (i) is provided by means of converting a biomass according to a gassing process.

9. Method according to claim 7, wherein the first gas mixture according to step (i) is provided by means of converting a hydrocarbon-containing gas mixture according to a steam reforming process.

10. Method according to any one of the claims 1 - 6, wherein prior to introduction into the at least one vessel (1, 2, 3, 4) the value of the pressure (*p*₁) of the second gas mixture (A/B)₂ is increased by a factor of at least 10 to a value of *p*₂ (*p*₂ ≥ 10 *p*₁), preferably by a factor of at least 30 (*p*₂ ≥ 30 *p*₁), more preferably by a factor of at least 50 (*p*₂ ≥ 50 *p*₁).

11. Method according to any one of the preceding claims, wherein the step (ii) of separating the first gas mixture (A/B)₁ is carried out according to a washing process.

12. Method according to any one of the claims 1 - 10, wherein the step (ii) of separating the first gas mixture (A/B)₁ is carried out by means of a membrane.

13. Method according to any one of the claims 1 - 7, wherein the step (ii) of separating the first gas mixture (A/B)₁ is carried out according to a pressure swing adsorption process (PSA process).
